# EUROPEAN PATENT SPECIFICATION

(11) **EP 3 331 439 B1**
(45) Date of publication and mention of the grant of the patent: **19.07.2023**
(21) Application number: 16833989.3
(22) Date of filing: 06.08.2016
(51) Int. Cl.: A61B 5/08, A61B 5/0205, A61B 5/024, A61B 5/087, A61B 5/1455, A61B 5/00

(54) **METHODS AND DEVICES FOR MONITORING RESPIRATION USING PHOTOPLETHYSMOGRAPHY SENSORS**
VERFAHREN UND VORRICHTUNGEN ZUR ÜBERWACHUNG DER BEATMUNG MITHILFE VON PHOTOPLETHYSMOGRAPHIESENSOREN
PROCÉDÉS ET DISPOSITIFS DE SURVEILLANCE RESPIRATOIRE UTILISANT DES CAPTEURS DE PHOTOPLÉTHYSMOGRAPHIE

(30) Priority: 06.08.2015 US 201562202141 P; 17.09.2015 US 201562220143 P; 08.10.2015 US 201562239148 P
(43) Date of publication of application: 13.06.2018
(73) Proprietor: Xhale Assurance, Inc., Gainesville, FL 32608 (US)
(72) Inventor: SHIAU, Deng-shan, Gainesville, FL 32653 (US)
(74) Representative: Philips Intellectual Property & Standards
(86) International application number: PCT/US2016/045930
(87) International publication number: WO 2017/024293

(56) References cited:
- WO-A1-00/21438
- WO-A1-2015/106280
- US-A- 5 190 048
- US-A1- 2009 323 049
- US-A1- 2011 112 442
- US-A1- 2011 245 628
- US-A1- 2015 105 632

## Description

### FIELD OF THE INVENTION

The present invention relates to biological sensors, and in particular, to the use of biological sensors to monitor physiological parameters of individuals. The present invention also relates to methods of processing data from biological sensors.

### BACKGROUND OF THE INVENTION

Photoplethysmography, or "PPG", is an optical technique for detecting blood volume changes in a tissue. In this technique, one or more emitters are used to direct light at a tissue and one or more detectors are used to detect the light that is transmitted through the tissue ("transmissive PPG") or reflected by the tissue ("reflectance PPG"). The volume of blood, or perfusion, of the tissue affects the amount of light that is transmitted or reflected. Thus, the PPG signal varies with changes in the perfusion of the tissue.

The blood volume in a tissue changes with each heartbeat, and so the PPG signal also varies with each heartbeat. Traditionally, this component of the PPG signal is referred to as the "AC component" of the signal, and is also often referred to as the "pulsatile component." Blood volume is also affected by other physiological processes in the body, including respiration, venous blood volume, sympathetic and parasympathetic tone and certain pathologies. The changes in the PPG signal due to these and other physiological processes, along with changes in the PPG signal due to noise caused by non-physiological processes such as ambient light and bodily movement, have traditionally been referred to collectively as the "DC component."

Pulse oximetry is a well-known physiological monitoring tool whereby PPG is used to monitor arterial blood oxygen saturation (SpO₂) in an individual. In typical pulse oximetry, red and IR radiation is transmitted through a tissue of the individual. The amplitude of the AC component of the PPG signal for IR and red wavelengths is sensitive to changes in SpO₂ due to the difference in the light absorbance of oxygenated and deoxygenated hemoglobin at these wavelengths. From their amplitude ratio, using the DC components to normalize their respective signals, the SpO₂ can be estimated. Traditionally, pulse oximetry has been performed at peripheral sites, but in recent years, alternative monitoring sites have been investigated, including at the nose (*e.g.,* septum, nasal alar), ear (*e.g.,* earlobe, concha), and forehead.

The nose, in particular the nasal alar, has recently been identified as a particularly promising site for PPG. Traditional sites for monitoring PPG, such as fingers and toes, generally provide a relatively small PPG signal, and the quality of this signal may be negatively impacted by sympathetic innervation in these tissue sites. Thus, in some cases, pulse oximetry measurements at peripheral sites may be unavailable or unreliable. Furthermore, due to the small signal size, the DC component signal from traditional peripheral sites may not be of sufficient strength and quality to allow for the DC signal to be used to reliably monitor physiological processes.

The nasal alar region has been shown by the inventor to provide a very large PPG signal relative to other sites of the body, including the fingers, toes and ears, and a relatively high quality signal due to its lack of sympathetic innervation. The improved PPG signal at the nasal alar site has allowed for a number of physiological parameters, including respiration rate, blood flow, respiratory effort and venous capacitance to be effectively extracted from the DC signal. Examples of patents and applications that describe the use the nasal alar site to obtain PPG signals, as well as a description of parameters and physiological processes that may be extracted from such signals, include U.S. Patent Nos. 6,909,912, 7,127,278, 7,024,235, 7,785,262, 7,887,502, 8,161,971, 8,679,028 and 8,641,635.

US 2011/0245628 A1 discloses a method for processing a photoplethysmograph signal that comprises decomposing the PPG signal into intrinsic mode functions or frequency components. In one embodiment, an intrinsic mode function contains a waveform indicative of a patient's respiration.

WO 00/21438) discloses a method of determining a respiratory rate from an optoplethysmogram. The raw optoplethysmogram signal is processed and filtered to generate a processed signal containing primarily respiratory rate frequency components.

WO 2015/106280 A1 discloses a method for determining the effectiveness of respiration. The method proposes to compare PPG signals with signals from a secondary respiration sensor, which in one embodiment is a thermistor. Respiration can be determined from a PPG signal stream by using a zero-cross method.

### SUMMARY OF EMBODIMENTS OF THE INVENTION

Provided according to embodiments of the invention are computer-implemented methods of monitoring respiration in an individual. Such methods may include obtaining a PPG signal stream from a central source site of the individual; identifying peaks and troughs of the PPG signal stream within a predetermined epoch, wherein a time between peaks or a time between troughs corresponds to a heart rate of the individual; determining significant local maxima of peak amplitudes and significant local minima of trough amplitudes within the predetermined epoch, wherein each significant local maxima of peak amplitude corresponds to an onset of the individual's respiratory effort to exhale and each significant local minima of trough amplitude corresponds to an onset of the individual's respiratory effort to inhale; calculating the number of significant local maxima, significant local minima, or both, during the predetermined epoch to determine the number of respiratory efforts in the predetermined epoch; and displaying the number of respiratory efforts in the predetermined epoch on a computer monitor.

In some embodiments of the invention, methods include determining the number of ventilations of the individual during the predetermined epoch. In some embodiments of the invention, the thermistor data is analyzed by a method comprising identifying significant monotonic decreases in the thermistor signal stream that occur for a time longer than a preset time period. In some cases, identifying significant monotonic decreases in the thermistor signal includes identifying initial monotonic decreases in the thermistor signal stream that occur for a time longer than a preset time period; rejecting the initial periods of a monotonic decrease having an amplitude decrease of less than a preset value; and designating the remaining initial periods of monotonic decrease as significant periods of monotonic decrease. In some cases, the number of ventilations and the number of respiratory efforts during the predetermined epoch are used to determine whether the individual has apnea.

In some embodiments of the invention, the evaluation to determine whether an apneic or hypopneic event has occurred includes determining a signal variation of the amplitude in each time interval that exceeds the predefined time limit; and if a time interval has a signal variation of greater than or equal to a first predefined value (c1), then the time interval is deemed to not include an apneic or hypopneic event; and if a time interval has a signal variation of less than the first predefined value (c1), then the time interval is deemed to include an apneic or hypopneic event.

In some embodiments, if a predetermined number of hypopneic and/or apneic events are indicated by the computer, a predetermined action is effected. For example, in some cases, the predetermined action includes at least one of initiate an alarm, rouse the individual, administer or increase oxygen supply to the individual or administer a narcotic reversal agent to the individual.

### BRIEF DESCRIPTION OF THE FIGURES

**Figure** 1 is a photoplethysmograph showing peaks and troughs that correspond to the individual's heart beat.
**Figure** 2 is a photoplethysmograph showing peaks and troughs that correspond to the individual's heart beat.
**Figure** 3 is a photoplethysmograph showing peaks and troughs that correspond to the individual's heart beat.
**Figure** 4 is a thermistor signal stream according to an embodiment of the invention.
**Figure** 5 is a flow chart summarizing a respiratory algorithm according to an embodiment of the invention.
**Figure** 6 is a flow chart summarizing a respiratory algorithm according to an embodiment of the invention.
**Figure** 7 is a flow chart summarizing a respiratory algorithm according to an embodiment of the invention.
**Figure** 8 is a thermistor signal stream according to an embodiment of the invention.
**Figure** 9 is a flow chart summarizing a respiratory algorithm according to an embodiment of the invention.
**Figure 10** is a flow chart summarizing a respiratory algorithm according to an embodiment of the invention.

### DETAILED DESCRIPTION OF EMBODIMENTS OF THE INVENTION

The present invention now will be described more fully hereinafter with reference to the accompanying drawings, in which embodiments of the invention are shown. However, this invention should not be construed as limited to the embodiments set forth herein. Rather, these embodiments are provided so that this disclosure will be thorough and complete, and will fully convey the scope of the invention to those skilled in the art.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof. As used herein, the term "and/or" includes any and all combinations of one or more of the associated listed items.

It will be understood that when an element is referred to as being "connected" or "coupled" to another element, it can be directly connected or coupled to the other element or intervening elements may be present. In contrast, when an element is referred to as being "directly connected" or "directly coupled" to another element, there are no intervening elements present. Like numbers refer to like elements throughout the specification. It will be understood that, although the terms first, second, etc. may be used herein to describe various elements, these elements should not be limited by these terms. These terms are only used to distinguish one element from another. Thus, a first element discussed below could be termed a second element without departing from the teachings of the present invention.

Provided according to embodiments of the present invention are computer-implemented methods of monitoring respiration in an individual using a photoplethysmography (PPG) signal secured to a central source site, and, optionally, at least one additional secondary respiration sensor. As used herein, an individual, also referred to as a patient, includes any mammal, including humans of any age. The individual may be monitored in any care setting including, but not limited to, hospitals (e.g., operating room (OR), intensive care unit (lCU), general care floors, or during transport therein), nursing homes, medical offices, medical transport and homes. The individual may be intubated or may be spontaneously breathing.

### Detection of Respiratory Efforts Using PPG Monitoring

In embodiments of the invention described herein, the PPG sensor may be secured to any central source site, and in particular, to a portion of the nose (e.g., the nasal alar or nasal septum). However, in particular embodiments, the PPG sensor is secured to the nasal alar of the individual. The term "secure" means to attach sufficiently to the skin to allow for a suitable PPG signal to be generated. In some cases, the sensor body is configured to secure onto the skin such that no additional support is necessary to allow for a suitable PPG signal to be reliably generated. However, in some cases, the sensor may be secured with the aid of an external support, for example, an additional structural support, a wire or cord, or an adhesive product such as tape. Such supports may be desirable to stabilize the sensor to prevent against signal loss, for example, due to the patient's movement (e.g., shivering), or due to movement (e.g., jostling, pulling, pushing) of the sensor or a cable attached thereto.

The PPG sensors include one or more components that emit light, and such components will be referred to herein as "emitters." As used herein, the term "light" is used generically to refer to electromagnetic radiation, and so the term includes, for example, visible, infrared and ultraviolet radiation. Any suitable type of emitter may be used, but in some embodiments, the emitter is a light-emitting diode (LED). In particular embodiments, a first emitter emits light at a first wavelength, and a second emitter emits light at a second wavelength. In some cases, a single emitter may emit light at a first wavelength and a second wavelength. One or more photodetectors, also referred to as "detectors", are also included in the PPG sensor. The detector is configured to detect light from an emitter, and this detected light generates a PPG signal. Any suitable photodetector may be used. However, examples of photodetectors include photodiodes, photoresistors, phototransistors, light to digital converters, and the like.

The computer-implemented methods described herein include of one or more of the steps of: (1) identifying peaks and troughs of a PPG signal stream within a predetermined epoch, wherein the peaks (or troughs) occur at a heart rate of the individual; (2) determining significant local maxima of the amplitudes of the peaks ("peak amplitudes") and significant local minima of the amplitudes of the troughs ("trough amplitudes") within the predetermined epoch, wherein each significant local maximum of peak amplitudes corresponds to the onset of the individual's respiratory effort to exhale and each significant local minimum of trough amplitudes corresponds to the onset of the individual's respiratory effort to inhale; (3) calculating the number of significant local maxima, the significant local minima, or both, during the predetermined epoch to determine the number of respiratory efforts during the predetermined epoch; and optionally, (4) displaying the number of respiratory efforts in the predetermined epoch on a computer monitor, or using the number of respiratory efforts per epoch for further analysis of the individual's respiration.

As used herein, the "PPG signal stream" is the waveform that is generated from the PPG sensor, and the "predetermined epoch" is any interval of time, for example, 10 seconds, 30 seconds, 1 minute, 2 minutes, etc. In some cases, the predetermined epoch is in the range of 10 seconds to 1 minute. This epoch provides a standard time period for counting respiratory efforts (e.g., RE per minute), and in some cases, ventilations, of the individual so that changes in respiration can be monitored over time.

As used herein, the term "respiratory effort" is meant to refer to an attempt by the individual to take a breath, whether or not ventilation occurs. Respiratory efforts imply that the muscles of respiration are contracting in response to signals from the brainstem. The degree of contraction of the respiratory muscles determines the tidal volume (V_{T}) when the airway is patent. If airway obstruction occurs, muscle contraction may result in decreased V_{T} or in the case of complete obstruction, no airway movement despite muscle contraction. Numerous brainstem inputs including the arterial oxygen saturation (PₐO₂), arterial CO₂ (PₐCO₂) and inputs from various receptors in the respiratory muscles determine the degree of contraction of the respiratory muscles. Disease states (CNS and non-CNS), medications (e.g. opioids, benzodiazepines, etc.) and other inputs may alter the "gain" of the brainstem and may decrease or prevent contraction of the respiratory muscles. As the PPG signal may be modulated in response to changes in intrathoracic pressure, including the changes in intrathoracic pressure due to respiratory effort-related muscle contractions, PPG may be used to monitor respiratory effort. The significant local minima of trough amplitudes of the PPG signal correlate to the onset of the individual's attempt to inhale and the significant local maxima of the peak amplitudes correlate to the onset of the individual's attempt to exhale.

The PPG sensor transmits raw PPG signals to a signal processing device, which will be discussed in further detail below. In some cases, the raw PPG signals are "conditioned" or filtered or smoothed before being analyzed to determine respiratory efforts. In general, such conditioning is achieved by band pass filters, which may filter out undesirably high or low frequency noise in the signal. While in some embodiments, the PPG signal from only one wavelength of light is processed and/or monitored, in other embodiments, PPG signals may be obtained from two or more wavelengths of light (*e.g.,* IR and red wavelengths) and in some cases, comparing the PPG signal (or respiratory efforts per epoch calculated therefrom) at different wavelengths may be useful.

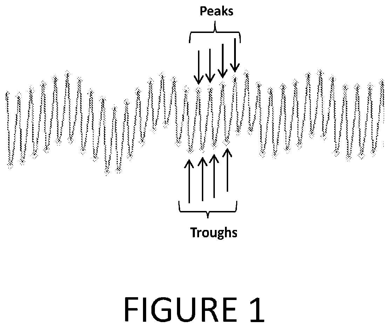

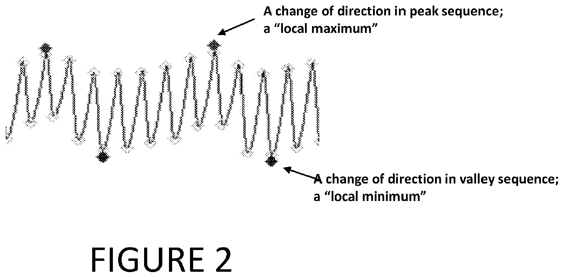

The peaks and troughs that correspond to the individual's heart beat are shown, for example, in **Figure 1****.** The amplitude of the peaks and troughs are monitored over time. As used herein, the amplitude is measured from a center or mean of the waveform. The "local maxima" and "local minima" of the amplitudes of the peaks ("peak amplitudes") and troughs ("trough amplitudes), respectively, are identified by a change of direction in the instantaneous rate of change (slope) of the peak and trough amplitudes over time. For example, a local maximum of peak amplitudes occurs when the instantaneous rate of change of peak amplitudes changes from positive to negative, and a local minimum of trough amplitudes occurs when the instantaneous rate of change of trough amplitudes changes from negative to positive. See **Figure 2****.**

A local minimum or local maximum may be deemed a significant local maximum or significant local minimum, respectively, at the outset, or the algorithm in the signal processor may determine initial local maxima and initial local minima, and designate additional criteria that must be met before the initial local maxima or minima is deemed a significant local maxima or minima, respectively. For example, the algorithm may reject an initial local maximum if the local maximum deviates in amplitude from at least one preceding peak by less than a preset value. Alternatively, or additionally, the algorithm may reject an initial local maximum if the local maximum deviates in amplitude from at least one peak immediately following it by less than a preset amount. Thus, a local maximum that is very similar in amplitude to the peak(s) immediately before and/or after it may be rejected as not being a significant local maximum because it is not sufficiently distinct (in amplitude) from the peak(s) immediately before and/or after it. The algorithm may also reject an initial local minimum in the same manner. If a local minimum deviates in amplitude from at least one trough immediately preceding it and/or at least one trough immediately following it by less than a preset amount, it may be rejected as not being a significant local minimum. Thus, a local minimum that is very similar in amplitude to the trough(s) immediately before and/or after it may be rejected as not being a significant local minimum because it is not sufficiently distinct in amplitude from the trough(s) immediately before and/or after it.

The computer/signal processor calculates the number of significant local maxima, the number of significant local minima, or both, during the epoch to determine the number of respiratory efforts during the epoch. Each respiratory effort is a combined inhalation and exhalation, and so the computer can calculate the respiratory efforts from the number of significant local maxima, the number of significant local minima, or both. If both the significant local maxima and significant local minima are used, the values obtained by each may be compared and evaluated to provide further confidence in the determinations. In particular embodiments, the significant local minima are counted to determine the number of respiratory efforts in the predetermined epoch. The respiratory efforts per epoch can then be displayed on a computer monitor or screen to guide the medical care of the individual, or the number of respiratory efforts per epoch may be used for further analysis of the individual's respiration, as described below. The calculation may be performed on a rolling basis so that the number of respiratory efforts per epoch is updated regularly, such as, for example, every 1, 2, 5, 10, 15 or 30 seconds.

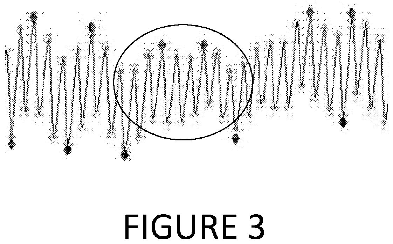

In some embodiments of the invention, the algorithm in the signal processor may have an additional step to detect onset of inhalations and onset of exhalations that were not detected as significant local maxima or significant local minima, respectively, via the methods described above. In some cases, before the number of respiratory efforts during the epoch is calculated, the algorithm evaluates whether there are any missed onset of inhalations and/or any missed onset of exhalations. For example, referring to **Figure 3****,** in some cases the algorithm analyzes whether there is a missing onset of inhalation (missing local minima) by noting portions of the PPG signal stream where two or more significant local maxima occur without any local minima occurring at a time there between. In this case, the algorithm will count a missed onset of inhalation as having occurred, and therefore if the significant local minima are used to calculate respiratory efforts in the epoch, then this missed onset of inhalation adds an additional respiratory effort to the count. Conversely, in some embodiments, the algorithm may further analyze whether there is a missing onset of exhalation (missing local maxima) by noting portions of the signal stream where two or more significant local minima occur without any local maxima occurring at a time there between (not shown in figures). In this case, the algorithm will count a missed onset of exhalation as having occurred, and thus, if the significant local maxima are used to calculate respiratory efforts in the epoch, then this missed onset of exhalation adds an additional respiratory effort to the count.

In some embodiments of the invention, missed onset of inhalations or exhalations may alternatively or additionally be determined by other methods. For example, with respect to determining a missed onset of inhalation, in some cases, the algorithm may evaluate the waveform to determine portions of the PPG signal wherein a local maximum occurs between two local minima and the local maximum is separated from each of the two local minima by more than a preset amount of time. If the two local minima are significantly separated from the significant local maximum, then the algorithm counts an additional significant local minimum as occurring there between. Thus, if the number of respiratory efforts determined during the epoch are determined using the significant local minima, then an additional respiratory effort will be added based on the missed onset of inhalation detection.

With respect to determining a missed onset of exhalation, in some cases, the algorithm may evaluate the waveform to determine portions of the PPG signal wherein a local minimum occurs between two local maxima and the local minimum is separated from the two local maxima by a preset amount of time. If the two local maxima are significantly separated from the significant local minimum, then the algorithm counts an additional significant local maximum as occurring there between. Thus, if the number of respiratory efforts determined during the epoch are determined using the significant local maxima, then an additional respiratory effort will be added based on the missed onset of exhalation detection.

### Detection of Ventilations by Secondary Respiration Detectors

As used herein, "ventilation" is meant to refer to air movement that results in exchange of CO₂ and oxygen in the individual. A "ventilation" may also be referred to herein as a "breath". Respiratory efforts may result in ventilation, but in some cases, such as when the individual has obstructive apnea, the individual may make respiratory efforts but due to the obstruction, no ventilation occurs.

In some embodiments of the invention, a secondary respiration sensor at the nose is used to determine actual ventilation by the patient. Secondary respiration sensors may be used to compare with the respiratory information obtained from the PPG sensor(s). Such sensors include, but are not limited to, nasal air flow sensors, nasal pressure sensors, capnometers, thermistors, acoustic sensors, differential pressure transducers, and the like. In some cases, both the PPG sensor(s) and the secondary respiration sensor(s) are situated at the nose, and in some cases, a single device or system (e.g., an array) may include both the PPG sensor(s) and the secondary respiration sensor(s).

In particular embodiments, the secondary respiration sensor detects respiratory airflow or temperature changes at the nostril, such as with a thermistor. For example, during inhalation, a thermistor placed at the nostril detects a relative decrease in temperature compared to exhalation since, in most situations, body temperature, and therefore exhaled breath temperature, is higher than ambient temperature. Thus, detection of changes in temperature may be a suitable means to determine respiratory air flow and therefore, if ventilation occurred. Air flow from one or both nostrils may be monitored and compared with the PPG information.

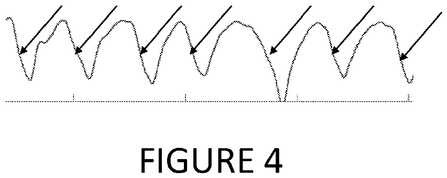

**Figure 4** shows a thermistor signal stream. The arrows point to monotonic decreases in slope of the signal due to a decrease in temperature detected during inhalation. In some embodiments of the invention, the computer-implemented methods described above may further include analyzing a thermistor signal stream obtained from nasal airflow of the individual during the predetermined epoch. Thus, the PPG signal stream and thermistor signal stream are synchronized chronologically and so each respiratory effort via PPG can be compared with thermistor data to see if the respiratory effort resulted in ventilation.

In some embodiments of the invention, the algorithm analyzes the thermistor signal stream to identify when significant signal decreases (e.g., monotonic decreases, such as those shown in **Figure 4**) occur for longer than a preset time period. The monotonic decreases longer than the preset time period are deemed to be inhalations that result in ventilation. Any suitable preset time period may be used. For example, in some cases, the time period is a range of 0.2 to 1 s, or in some cases 0.5s. As with the PPG signal stream, in some cases, the thermistor signal stream is band pass filtered, smoothed, or both, prior to identifying the significant periods of (monotonic) decrease.

In some cases, all monotonic signal decreases longer than the preset time period are deemed significant monotonic decreases. However, in some cases, the algorithm identifies initial monotonic decreases longer than a preset time and then rejects those monotonic decreases that have an amplitude decrease of less than a preset value, which in some embodiments is less than one standard deviation of the waveform for the epoch. The remaining initial monotonic decreases longer are then designated as significant monotonic decreases, and each significant monotonic decrease during the predetermined epoch is counted to determine the number of ventilations during the predetermined epoch. In other embodiments of the invention, in a first step, the algorithm first identifies monotonic decreases in amplitude that are greater than a preset value to arrive at the initial monotonic decreases. Then, the algorithm rejects initial monotonic decreases that decrease for less than a preset time period, and the remaining initial monotonic decreases are deemed significant monotonic decreases for the purposes of the methods described herein.

### Monitoring Respiration Using Respiratory Efforts & Ventilations

The PPG signal stream is thus used to determine the number of respiratory efforts during the predetermined epoch and the thermistor signal stream is used to determine the number of ventilations during the predetermined epoch. The two numbers can be compared to assess the quality of the individual's breathing. For example, if the number of respiratory efforts exceeds the number of ventilations by a certain percentage or by a certain number, the individual may be experiencing periods of obstructive apnea. In such cases, a "predetermined action" may be effected. For example, one of the following actions may be effected: an alarm may be initiated or medical personnel notified in some manner, the individual may be awaken, oxygen may be administered to the individual (via medical personnel or via a closed loop device) or oxygen flow may be increased, and narcotic reversal agents may be administered (automatically or by medical personnel) to the individual.

In some cases, the number of respiratory efforts and/or ventilations in the epoch may be compared with a preset value (such as, for example, 4 or 6 breaths per minute) and if either or both fall below the preset value, the individual may be considered to be in respiratory depression, and a predetermined action may be effected. In some cases, if either or both of the number of respiratory efforts and/or ventilations in the epoch are below the preset value, the algorithm may further analyze the blood oxygen saturation (SpO2) of the individual, and determine whether to effect a predetermined reaction based on the number of respiratory efforts and/or ventilations and the SpO2 of the individual. Furthermore, in some cases, the magnitude or rate of decrease of either the number of respiratory efforts and/or ventilations in the individual, the magnitude or rate of change in the SpO2, or both, may be used to determine whether to effect a predetermined reaction. For example, the algorithm may have as a condition that if the number of respiratory efforts and/or ventilations per epoch decreases by greater than 20%, the predetermined action is effected. In some cases, the algorithm may have as a condition that if the number of respiratory efforts and/or ventilations per epoch decreases by greater than 20%, and the SpO2 decreases below a predefined limit, the predetermined action is effected.

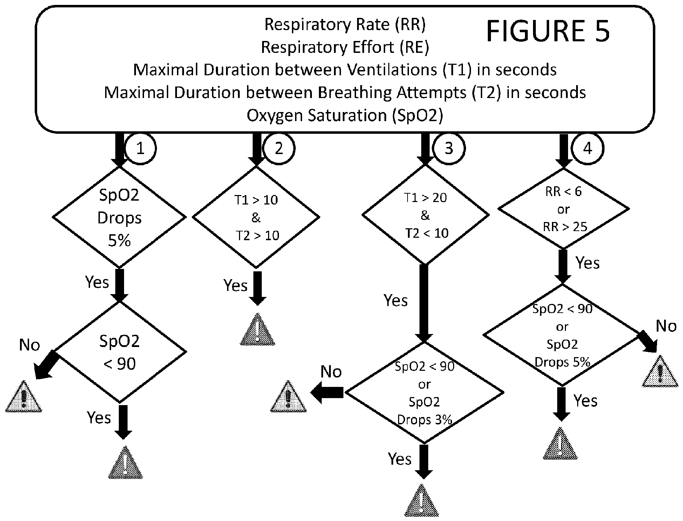

In addition or alternative to the methods described above, the signal processing device may also use other algorithms for respiratory monitoring as shown in **Figure 5****.** For example, pathway (1) shows a first algorithm based only on SpO2. If the SpO2 drops by a predefined amount or percentage (±5%) and the SpO₂ is below a predefined value (90%), then a predetermined reaction is effected (as identified by the red triangle). If the SpO2 drops by at least the predefined amount or percentage (±5%) but the SpO2 is still equal to or greater than the predefined value (90%), then a "warning" (visual, audible and/or only noted by the signal processing device) may be generated, but no alarm is sounded. The warning may cause no additional actions, or it may initiate a more rigorous level of analysis by the signal processing device. In **Figure 5****,** the warning is identified as the yellow triangle.

In the algorithm in pathway (2), the signal processing device detects the measurement between respiratory efforts and/or between ventilations. For T1, the maximal duration between ventilations during the epoch is determined by the time between significant monotonic decreases. The time of each monotonic decrease may be determined using any consistent point, such as the time at the start of the monotonic decreases, the time at the mean of the monotonic decrease, and the like. For T2, the maximal duration between respiratory efforts during the epoch is determined by the time between significant local maxima, significant local minima, or both. In the example in **Figure 5****,** if T1 and T2 are both greater than a predefined time period (and T1 and T2 can have different predefined time periods) in this case, 10 seconds, a predetermined action is effected.

In the algorithm illustrated in pathway (3) of **Figure 5****,** the signal processing device uses T1 and T2, as well as the SpO2. If T1 is greater than a predefined time (20s) and T1 is less than a predefined time (10s), and the SpO2 is less than a certain predefined value (90) or drops by at least a certain amount or percent (30%), then a predetermined action is effected. However, if T1 is greater than the predefined time (20s) and T1 is less than the predefined time (10s) but the SpO2 is equal to or greater than the predefined value (90) and does not drop by at least the certain amount or percentage (3%), then only a warning results.

In the algorithm illustrated in pathway (4) of **Figure 5****,** the signal processing device uses the number or respiratory efforts (RE) and the number of ventilations (RR) per epoch, as well as using the SpO2. In this example, if the RR is less than a predefined value (6) and the RE is greater than a predefined value (25), and the SpO2 is greater than a predefined value (90), or the SpO₂ drops by at least a certain amount or percentage (5%), then a predetermined reaction is effected. However, if the RR is less than a predefined value (6) and the RE is greater than a predefined value (25), but the SpO2 is greater than or equal to its predefined value, and the SpO2 has not dropped by at least the certain percentage or amount, then only a warning is indicated.

### Signal Processing

The PPG signals and secondary respiration signals are analyzed and algorithms are performed in any suitable fashion, but are typically monitored with a signal processing device, such as a general-purpose microprocessor, a digital signal processor (DSP) or application specific integrated circuit (ASIC). The singular term "signal processing device" may include two or more individual signal processing devices. Such signal processing devices may be adapted to execute software, which may include an operating system and one or more applications, as part of performing the functions described herein. In electronic communication with the signal processing device may be a computer memory, such as a read-only memory (ROM), random access memory (RAM), and the like. Any suitable computer-readable media may be used in the system for data storage. Computer-readable media are capable of storing information that can be interpreted by microprocessor. This information may be data or may take the form of computer-executable instructions, such as software applications, that cause the signal processing device to perform certain functions and/or computer-implemented methods. Depending on the embodiment, such computer-readable media may include computer storage media and communication media.

Computer storage media may include volatile and non-volatile, removable and non-removable media implemented in any method or technology for storage of information such as computer-readable instructions, data structures, program modules or other data. Computer storage media may include, but is not limited to, RAM, ROM, EPROM, EEPROM, flash memory or other solid state memory technology, CD-ROM, DVD, or other optical storage, magnetic cassettes, magnetic tape, magnetic disk storage or other magnetic storage devices, or any other medium which can be used to store the desired information and which can be accessed by components of the system.

Systems and devices for effecting the methods described herein are also disclosed herein. Computers or other signal processing devices, as well as software configured to perform such methods are included according to some embodiments of the invention.

### Example 1

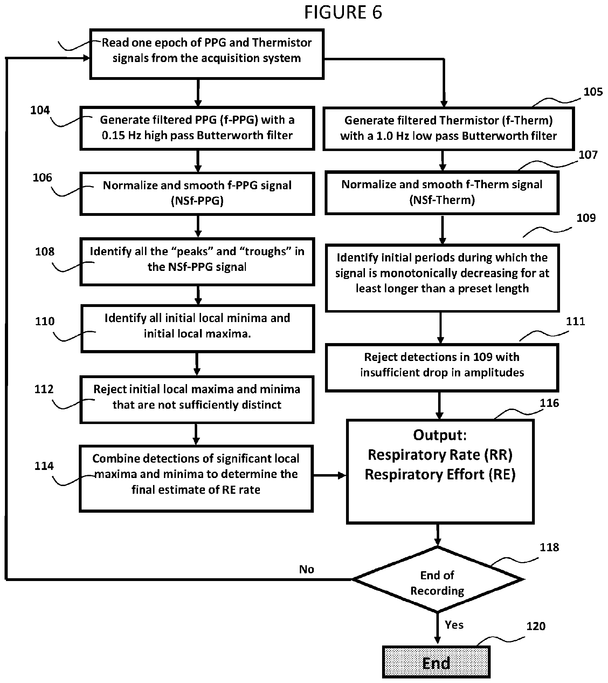

Referring to **Figure 6****,** in **Block 102,** the algorithm acquires one epoch of PPG and thermistor raw signals. In **Block 104,** to reduce the effects from the slow background shift unrelated to the respiratory effort (RE), the raw PPG signal is first high-pass filtered (f-PPG). As a specific example, the signal may be high-pass filtered at 0.15 Hz. In **Block 106,** to reduce the effects from the PPG amplitudes on detection of RE, the f-PPG signal is normalized (Nf-PPG). Furthermore, to reduce the high frequency noise (e.g., electrical noise), moving average (smoothing) is applied to the Nf-PPG signal (NSf-PPG). In **Block 108,** all the "peaks" and "troughs" in the NSf-PPG signal are identified (see **Figure 1****).**

Referring to **Figure 2****,** this is accomplished by searching all the local minimums and local maximums in the signal within the detection epoch. In **Block 110,** one of the most noticeable characteristics with respect to the PPG signal modulations due to RE is the instantaneous rate of change (i.e., the derivative) of the values of its peaks and troughs, respectively. Referring to **Figure 2****,** therefore, in this step, the algorithm searches for initial local maxima and initial local minima. Referring to **Block 112,** although (in **Block 104**) the algorithm has attempted to filter out the slow background activities in the PPG signal, there will still be peak or trough sequences that fit the patterns described in **Block 110** due to the slow background activities. However, in almost all cases, the non-RE-related modulations have much smaller variation of values within the sequence when compared to those due to RE. Therefore, in **Block 112,** the algorithm examines initial local maxima and/or minima detected in **Block 110** and rejects those with a standard deviation (within the sequence) smaller then a preset threshold to determine significant local maxima and/or significant local minima. Referring to **Block 114,** typically there is one modulation on peaks (caused by the onset of exhalation) after a modulation on trough (caused by the onset of inhalation). However, respiratory efforts do not always provide clear PPG modulations on both peaks and troughs. Sometimes a RE only causes modulation on peaks and sometimes only on troughs. Hence, to enhance the sensitivity of the detection, the algorithm checks for possible missed onset of inhalations and onset of exhalations based on the variability and the occurrences of the detections from both peaks and troughs. For instance, referring to **Figure 3****,** when there are two modulations on peaks between two modulations on troughs, the algorithm checks the time intervals between these modulations and determines if there is a missed detection of a RE.

Referring to **Block 105,** to reduce the effects from the high frequency noise, the raw thermistor signal is first low-pass filtered, for example, at 1.0 Hz (f-Therm). In **Block 107,** to reduce the effects from the thermistor signal amplitudes in detection of respiratory rate (RR), the f-Therm signal is normalized (Nf-Therm). Moving average (smoothing) is also applied to the Nf-Therm signal (NSf-Therm) to further reduce the high frequency noise. In **Block 109,** since during inspiration, the drop of the temperature results in the thermistor signal amplitude decrease, the algorithm searches for periods of NSf-Therm signal exhibiting monotonically decrease for at least longer than preset time length (see **Figure 4**) In **Block 111,** however, some monotonically decreasing periods (initial monotonic decreases) detected in **Block 109** may not have sufficiently large decrease in amplitudes to be considered an inspiration. Therefore, the algorithm rejects the periods detected in **Block 109** that have amplitude drop less than a preset amount to determine significant monotonic decreases in amplitude. In **Block 116,** after processing the PPG and thermistor signals for the given detection epoch, the algorithm generates an output box that displays RR and RE. In **Block 118,** after the detection process is finished for the current epoch, the system checks if the recording is ended, as depicted by **Block 118.** If the recording is finished, the system will be terminated, as illustrated by **Block 120.** If not, the system goes back to **Block 102** and reads and processes the next epoch of PPG and thermistor signals.

### Example 2

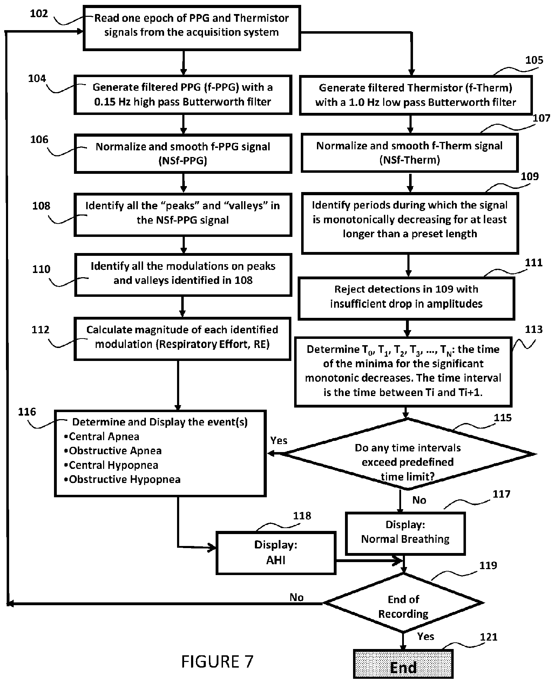

Also provided herein are algorithms that may be used in devices, systems and methods for the detection and classification of apneic and hypopneic, as shown in Figures 7-10, with further description below.

Referring to **Figure** 7, in **Block 102,** the algorithm acquires one predetermined epoch of PPG and thermistor raw signals. In **Block 104,** to reduce the effects from the slow background shift unrelated to the respiratory effort (RE), the raw PPG signal is first high-pass filtered (f-PPG). As a specific example, the signal may be high-pass filtered at 0.15 Hz. In **Block 106,** to reduce the effects from the PPG amplitudes on detection of RE, the f-PPG signal is normalized (Nf-PPG). Furthermore, to reduce the high frequency noise (e.g., electrical noise), moving average (smoothing) is applied to the Nf-PPG signal (NSf-PPG). In **Block 108,** all the "peaks" and "troughs" in the NSf-PPG signal are identified (see **Figure 1****).**

Referring to **Figure 1****,** this is accomplished by searching all the local minimums and local maximums in the signal within the predetermined epoch. In **Block 110,** one of the most noticeable characteristics with respect to the PPG signal modulations due to RE is the instantaneous rate of change (i.e., the derivative) of the values of its peaks and troughs, respectively. Referring to **Figure 2****,** therefore, in this step, the algorithm searches for initial local maxima and initial local minima. Referring to **Block 112,** the algorithm then quantifies the magnitude of the PPG modulations resulting in local maxima and/or minima to estimate the magnitude of the respiratory effort. For example, the difference between the amplitude of the local maxima and the peaks immediately preceding it (e.g., the 1,2,3 or 4 peaks preceding it) and/or the peaks immediately following it (e.g., the 1,2,3 or 4 peaks following it) may provide a measure of the modulation in the signal, and therefore the magnitude of the signal. Conversely, the difference between the amplitude of the local minima and the valleys immediately preceding it (e.g., the 1,2,3 or 4 peaks preceding it) and/or the peaks immediately following it (e.g., the 1,2,3 or 4 peaks following it) may also provide a measure of the modulation in the signal, and therefore the magnitude of the signal.

Referring to **Block 105,** to reduce the effects from the high frequency noise, the raw thermistor signal is first low-pass filtered, for example, at 1.0 Hz (f-Therm). In **Block 107,** to reduce the effects from the thermistor signal amplitudes in detection of respiratory rate (RR), the f-Therm signal is normalized (Nf-Therm). Moving average (smoothing) is also applied to the Nf-Therm signal (NSf-Therm) to further reduce the high frequency noise. In **Block 109,** since during inspiration, the drop of the temperature results in the thermistor signal amplitude decrease, the algorithm searches for periods of NSf-Therm signal exhibiting monotonically decrease for at least longer than preset time length (see **Figure 4**) In **Block 111,** however, some monotonically decreasing periods (initial monotonic decreases) detected in **Block 109** may not have sufficiently large decrease in amplitudes to be considered an inspiration. Therefore, the algorithm rejects the periods detected in **Block 109** that have amplitude drop less than a preset amount to determine significant monotonic decreases in amplitude.

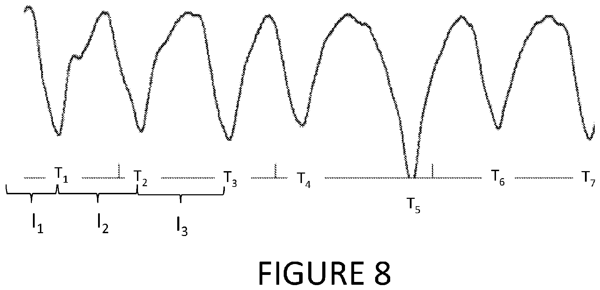

Referring to **Figure 8****,** the algorithm next records the time interval between significant monotonically decreasing periods in the predetermined epoch. For example, in some cases, the algorithm measures the time difference between successive minima (whereby each minima is associated with a significant period of monotonic decrease), as shown in **Figure 8****.** In this figure, the time between the start of the predetermined epoch and T₁ is the first time interval I₁, the time between T₁ and T₂ is the second time interval (I₂), etc. T₁, T₂, T₃, etc. are the successive minima of the significant monotonic decreases in the thermistor signal. The time between the last significant monotonic decrease in the predetermined epoch (T₇ in **Figure 8**) and the end of the predetermined epoch is the last time interval.

Referring back to **Figure 7****,** in **Block 115** the time intervals are evaluated to determine whether they exceed a predefined time limit. In a particular example, the predefined time limit is in a range between 5 and 15 seconds, in some cases, between 7 and 12 seconds and in some cases, the predefined time limit is 8, 9, 10, 11 or 12 seconds. In this particular example, the predefined time limit is 10 seconds. In doing this, the algorithm is determining whether respirations, as determined by significant monotonic decreases, are sufficiently far apart to suggest that an apneic or hypopneic event has occurred therebetween. Referring to **Block 117,** if no time intervals in the predetermined epoch exceed the predefined time limit, normal breathing is determined to have occurred. In some cases, a caregiver may be alerted to the fact that normal breathing is occurring (e.g., as shown on the computer monitor or with audible sounds) and in some cases, a determination of normal breathing may not be communicated. If normal breathing is determined to have occurred during the predetermined epoch, the algorithm checks whether the recording has ended, as shown in **Block 119.** If the recording has ended, the system will be terminated, as shown in **Block 121.** If recording has not ended, the algorithm proceeds again to **Block 102** and reads and processes the next predetermined epoch of PPG and thermistor signals.

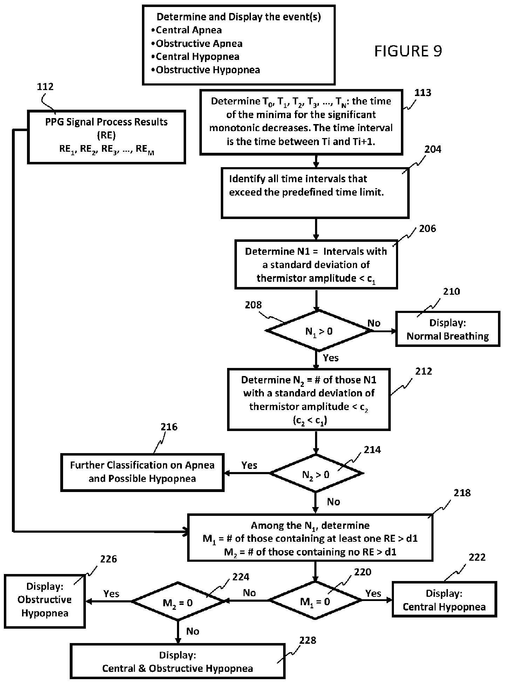

If in **Block 115,** any time intervals exceed the predefined time limit, such time intervals are further evaluated in **Block 116** to both determine whether an abnormal breathing event has occurred and to classify the event as a central apnea, obstructive apnea, central hypopnea or obstructive hypopnea. The details of this analysis are shown in **Figure 9. Figure 9** again shows **Block 113** whereby the time intervals between significant monotonic decreases in the thermistor signal are measured. In **Block 204,** the time intervals that exceed a predefined time limit are identified. Next, in **Block 206,** the algorithm determines N₁, the number of time intervals (I_{N1}) wherein the standard deviation of the amplitude of the thermistor signal is less than a predefined value, c₁. In some cases, the predefined value may be based on the standard deviation of the thermistor signal for a time period prior to the time interval. For example, the predefined value may be 50% of the standard deviation of the thermistor signal for the 30 seconds prior to the time interval. While in this example, the standard deviation is used as a measure of the signal variation, other measures of signal variation (e.g., range, variance, etc.) may be used.

Referring to **Block 208,** if none of the time intervals that exceed the predefined time limit have a standard deviation in amplitude less than the predefined value, c₁, N₁ is 0, and normal breathing is deemed to have occurred, as shown in **Block 210.** If at least one of the time intervals that exceeds the predefined time limit has a standard deviation in amplitude less than the predefined value, c1, then, as shown in Block **212,** the algorithm will further determine if the standard deviation of the amplitude of the thermistor signal in each of such N₁ intervals is also less than c₂, where c₂ < c₁. If so, the number of such time intervals is identified as N₂ and those intervals are defined as I_{N2}. In some cases, the predefined value c₂ may be based on the standard deviation of the thermistor signal for a time period prior to the time interval. For example, the predefined value c₂ may be 30% of the standard deviation of the thermistor signal for the 30 seconds prior to the time interval. While in this example, the standard deviation is used as a measure of the signal variation, other measures of signal variation (e.g., range, variance, etc.) may be used.

Analyzing the time intervals for those having a standard deviation of less than the first predefined value c₁ is meant to identify time intervals in which the signal does not vary greatly, so that while there may be small peaks and/or valleys, they are minor, and only a small amount of airflow is being detected by the thermistor during these time intervals. Analyzing the time intervals for those having a standard deviation of less than predefined value c₂ is meant to identify time intervals having even smaller peaks and/or valleys, so that they might be considered close to flat signals, and thus, essentially no nasal airflow is detected by the thermistor during these time intervals. Therefore, if a particular time interval has a standard deviation in amplitude of less than c₁ but greater than c₂ (the time interval is one of the N₁ intervals but not among those N₂), the algorithm determines that the time interval includes a hypopneic event. When a particular time interval has a standard deviation in amplitude of less than both c₁ and c₂ (the time interval belongs to both of those N₁ and N₂ intervals), the algorithm determines that the time intervals includes an apneic event.

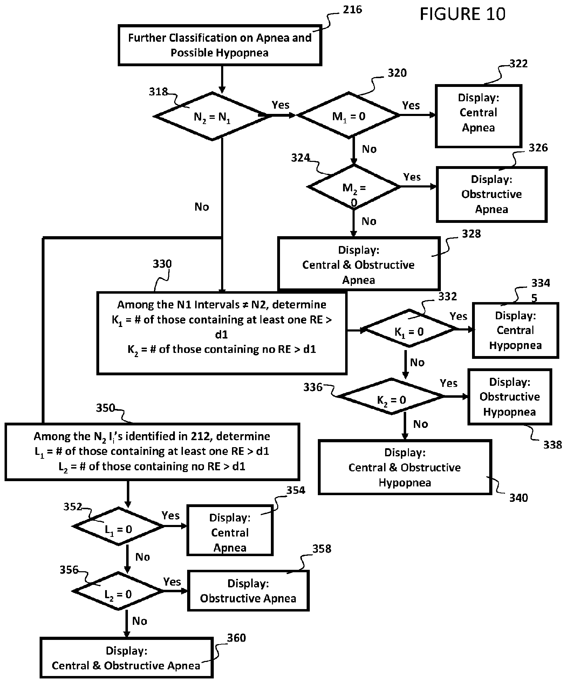

Thus, referring to **Block 214,** the algorithm may determine if any of the N₂ intervals are present in the predetermined epoch. If so, further analysis to determine the type of apnea (and possible hypopnea) may be performed, as shown in **Block 216,** which will be discussed further with reference to **Figure 10****.** If in **Block 214,** N₂ is determined to be zero, the algorithm proceeds to further analyze the N₁ time intervals by comparing the thermistor data with the corresponding PPG data (**Block 218**) to determine the type of hypopnea that has occurred.

As shown in **Block 218,** the algorithm looks at each of the N₁ intervals (that is not among N₂, which further defined as I_{N1}^{∗}) and identifies the corresponding portion of the PPG signal. If during any of the I_{N1}^{∗} time intervals, the magnitude of the respiratory effort (as determined by the magnitude of the modulation of the significant local maxima or minima) is greater than a predefined value d₁, then the algorithm designates this I_{N1}^{∗} as an I_{M1}, which denotes that an obstructive event has occurred. If any I_{N1}^{∗} time intervals in the predetermined epoch have a respiratory effort of less than predefined value d₁, then these time intervals are designated as I_{M2}. Thus, for any given predetermined epoch, if there are no I_{M1} present, (see **Block 220**), then central hypopnea (**Block 222**) is deemed to have occurred. If there is at least one I_{M1} present, the algorithm evaluates if any I_{M2} are also present (**Block 224**). If I_{M2} are present along with I_{M1} in the predetermined epoch, then central and obstructive hypopnea are deemed to have both occurred (**Block 228**). If only I_{M1} time intervals are present (no I_{M2} present), obstructive hypopnea is deemed to have occurred (**Block 226**).

As discussed above, if in **Block 214** of **Figure 9****,** any I_{N2} are present, apneic, and possibly also hypopneic, events are deemed to have occurred. To determine the types of apneic (and possibly hypopneic) events, the algorithm then follows the analysis summarized in **Figure 10****.** Referring to **Block 318,** if every I_{N1} is also an I_{N2}, then every time interval in the pretermined epoch that exceeds the predefined time limit has an extremely small variation in amplitude. Thus, only apnea is deemed to have occurred, and the PPG signal will then be compared with the thermistor signal to determine the type of apnea. If during a I_{N2} time interval, the magnitude of the respiratory effort (as determined by the magnitude of the modulation of the significant local maxima or minima) is greater than predefined value d₁, the algorithm designates this I_{N2} as an I_{M1}, which denotes that an obstructive event has occurred. If any I_{N2} time intervals in the predetermined epoch have a respiratory effort of less than predefined value d₁, then these time intervals are designated as I_{M2}. Thus, for any given predetermined epoch, if there are no I_{M1} present, (see **Block 320**), then central apnea (**Block 322**) is deemed to have occurred. If there is at least one I_{M1} present, the algorithm evaluates if any I_{M2} are also present (**Block 324**). If I_{M2} are present along with I_{M1} in the predetermined epoch, then central and obstructive apnea are deemed to have both occurred (**Block 328**). If only I_{M1} time intervals are present (no I_{M2} present), obstructive apnea is deemed to have occurred (**Block 326**).

Referring back to **Block 318,** in the event that not every I_{N1} is also an I_{N2}, then the predetermined epoch is deemed to include both apneic and hypopneic events. The apneic and hypopniec events are evaluated separately. The time intervals in the predetermined epoch wherein I_{N1} is also I_{N2} are deemed to indicate apnea, and the analysis follows a pathway similar to that in **Blocks 320-326.** Referring to **Block 350,** if during a I_{N2} time interval, the magnitude of the respiratory effort (as determined by the magnitude of the modulation of the significant local maxima or minima) is greater than predefined value d₁, the algorithm designates this I_{N2} as an I_{L1}, which denotes that an obstructive event has occurred. If any I_{N2} time intervals in the predetermined epoch have a respiratory effort of less than predefined value d₁, then these time intervals are designated as I_{L2}. Thus, for any given predetermined epoch, if there are no I_{L1} present, (see **Block 352**), then central apnea (**Block 354**) is deemed to have occurred. If there is at least one I_{L1} present, the algorithm evaluates if any I_{L2} are also present (**Block 356**). If I_{L2} are present along with I_{L1} in the predetermined epoch, then central and obstructive apnea are deemed to have both occurred (**Block 360**). If only I_{L1} time intervals are present (no I_{L2} present), obstructive apnea is deemed to have occurred (**Block 358**).

The time intervals in the predetermined epoch wherein I_{N1} is not I_{N2} are deemed to indicate hypopnea, and the analysis follows a pathway similar to that in **Blocks 320-326.** Referring to **Block 330,** if during a I_{N1} time interval (that is not also I_{N2}), the magnitude of the respiratory effort (as determined by the magnitude of the modulation of the significant local maxima or minima) is greater than predefined value d₁, the algorithm designates this I_{N1} as an I_{K1}, which denotes that an obstructive event has occurred. If any I_{N1} time intervals in the predetermined epoch have a respiratory effort of less than predefined value d₁, then these time intervals are designated as I_{K2}. Thus, for any given predetermined epoch, if there are no I_{K1} present, (see **Block 332**), then central hypopnea (**Block 334**) is deemed to have occurred. If there is at least one I_{K1} present, the algorithm evaluates if any I_{K2} are also present (**Block 336**). If I_{K2}are present along with I_{K1} in the predetermined epoch, then central and obstructive hypopnea are deemed to have both occurred (**Block 340**). If only I_{K1} time intervals are present (no I_{K2} present), obstructive hypopnea is deemed to have occurred (**Block 338**).

Referring to **Figure 7****,** once the algorithm completes the identification and classification analyses in **Block 116,** the algorithm calculates an **Apnea-Hypopnea Index (AHI),** as depicted in **Block 118.** The AHI can be calculated as the total number of Apnea/Hypopnea events from the beginning of the study, the rate of the event occurrences per time period (e.g., hour), or both. The AHI may be displayed, for example, on a computer monitor to alert a caregiver. In some embodiments, if a certain number of apneic and/or hypopneic events occur, or if the AHI exceeds a predefined value, a predetermined reaction may be effected. For example, one or more of the following predetermined reactions may be effected: an alarm may be initiated, the individual may be roused, the administration or increase in oxygen supply may be initiated, and a narcotic reversal agent may be administered.

In the specification, there have been disclosed embodiments of the invention and, although specific terms are employed, they are used in a generic and descriptive sense only and not for purposes of limitation, the scope of the invention being set forth in the following claims.

## Claims

1. A computer-implemented method of monitoring respiration in an individual comprising
obtaining (102) a PPG signal stream from a central source site of the individual;
identifying (108) peaks and troughs of the PPG signal stream within a predetermined epoch, wherein a time between peaks or a time between troughs corresponds to a heart rate of the individual;
determining (110, 112) significant local maxima of peak amplitudes and significant local minima of trough amplitudes within the predetermined epoch, wherein each significant local maxima of peak amplitude corresponds to an onset of the individual's respiratory effort to exhale and each significant local minima of trough amplitude corresponds to an onset of the individual's respiratory effort to inhale;
calculating (114) the number of significant local maxima, significant local minima, or both, during the predetermined epoch to determine the number of respiratory efforts in the predetermined epoch; and
displaying (116) the number of respiratory efforts in the predetermined epoch on a computer monitor,
**characterized in that**
determining significant local maxima of peak amplitudes comprises
determining an amplitude of each peak in the PPG signal stream during the predetermined epoch;
determining an increase or decrease in successive peaks in the PPG signal stream to identify initial local maxima;
rejecting (112) initial local maxima that deviate in amplitude from at least one immediately preceding and/or immediately following peak by less than a preset threshold; and
designating remaining initial local maxima as significant local maxima, and wherein determining significant local minima of trough amplitudes comprises
determining an amplitude of each trough in the PPG signal stream during the predetermined epoch;
determining an increase or decrease in successive troughs in the PPG signal stream to identify initial local minima;
rejecting (112) initial local minima that deviate from at least one immediately preceding and/or immediately following trough by less than a preset threshold; and
designating remaining initial local minima as significant local minima.

2. The computer-implemented method of monitoring respiration of claim 1, wherein determining the number of respiratory efforts in the predetermined epoch further comprises counting missed onset of inhalations, missed onset of exhalations, or both;
wherein a missed onset of exhalation is determined to have occurred when (a) two or more significant local minima occur without a significant local maxima occurring at a time there between, or (b) a local maximum occurs between two local minima and the local maximum is separated from each local minimum by more than a preset amount of time, and
wherein a missed onset of inhalation detection is determined to have occurred when (a) two or more significant local maxima occur without a significant local minima occurring at a time there between, or (b) a local minimum occurs between two local maxima and the local minimum is separated from each local maxima by more than a preset amount of time.

3. The computer-implemented method of monitoring respiration of claim 1, further comprising determining the number of ventilations of the individual during the predetermined epoch.

4. The computer-implemented method of claim 3, wherein the number of ventilations is determined by analyzing a thermistor signal stream obtained from nasal airflow of the individual during the predetermined epoch by a method comprising identifying (107, 109) significant monotonic decreases in the thermistor signal stream that occur for a time longer than a preset time period, and wherein the number of ventilations in the predetermined epoch is the number of significant monotonic decreases in the thermistor signal during the predetermined epoch, wherein identifying significant monotonic decreases in the thermistor signal comprises:
identifying (109) initial monotonic decreases in the thermistor signal stream that occur for a time longer than a preset time period;
rejecting (111) the initial monotonic decreases having an amplitude decrease of less than a preset value; and
designating the remaining initial monotonic decreases as significant monotonic decreases.

5. The computer-implemented method of claim 1, wherein if the number of respiratory efforts is lower than a predetermined value, a predetermined action is effected, such predetermined action comprising at least one of initiate an alarm or rouse the individual.

6. The computer-implemented method of claim 1, wherein the magnitude of the significant local maxima, significant local minima, or both are evaluated to determine the magnitude of the respiratory efforts in the predetermined epoch.

7. The computer-implemented method of claim 4, further comprising determining time intervals between significant monotonic decreases in the thermistor signal, by:
determining an amplitude minimum for each significant monotonic decrease in the predetermined epoch;
measuring the time interval (a) between each successive amplitude minimum of each significant monotonic decrease, (b) between the start of the predetermined epoch and the first amplitude minimum; and (c) between the last amplitude minimum and the end of the predetermined epoch, to thereby determine the time intervals between significant monotonic decreases in the thermistor signal.

8. A computer-readable medium that stores computer-executable instructions that, when interpreted by executed by a signal processing device, cause the signal processing device to perform the computer-implemented method of any of claims 1 to 7.

9. A signal processing device configured to perform the computer-implemented method of any of claims 1 to 7.

## Patentansprüche

1. Computerimplementiertes Verfahren zu der Überwachung von Atmung bei einem Individuum, umfassend
Erlangen (102) eines PPG-Signalstroms von einem zentralen Quellort des Individuums;
Identifizieren (108) von Spitzen und Tälern des PPG-Signalstroms innerhalb eines vorbestimmten Zeitraums, wobei eine Zeit zwischen Spitzen oder eine Zeit zwischen Tälern einer Herzfrequenz des Individuums entspricht; Bestimmen (110, 112) signifikanter lokaler Maxima von Spitzenamplituden und signifikanter lokaler Minima von Talamplituden innerhalb des vorbestimmten Zeitraums, wobei jedes signifikante lokale Maximum der Spitzenamplitude einem Einsetzen der Atmungsanstrengung des Individuums zum Ausatmen entspricht und jedes signifikante lokale Minimum der Talamplitude einem Einsetzen der Atmungsanstrengung des Individuums zum Einatmen entspricht;
Berechnen (114) der Anzahl signifikanter lokaler Maxima, signifikanter lokaler Minima oder beider während des vorbestimmten Zeitraums, um die Anzahl von Atmungsanstrengungen in dem vorbestimmten Zeitraum zu bestimmen; und
Anzeigen (116) der Anzahl von Atmungsanstrengungen in dem vorbestimmten Zeitraum auf einem Computerbildschirm, **dadurch gekennzeichnet, dass**
ein Bestimmen signifikanter lokaler Maxima von Spitzenamplituden ein Bestimmen einer Amplitude von jeder Spitze in dem PPG-Signalstrom während des vorbestimmten Zeitraums umfasst;
ein Bestimmen einer Zunahme oder Abnahme aufeinander folgender Spitzen im PPG-Signalstrom, um anfängliche lokale Maxima zu identifizieren;
Verwerfen (112) anfänglicher lokaler Maxima, die in der Amplitude von mindestens einer unmittelbar vorangehenden und/oder unmittelbar folgenden Spitze um weniger als einen voreingestellten Schwellenwert abweichen; und
bestimmen von verbleibenden anfänglichen lokalen Maxima als signifikante lokale Maxima, und wobei ein Bestimmen der signifikanten lokalen Minima von Talamplituden Folgendes umfasst
bestimmen einer Amplitude von jedem Tal in dem PPG-Signalstrom während des vorbestimmten Zeitraums;
bestimmen einer Zunahme oder Abnahme in aufeinanderfolgenden Tälern in dem PPG-Signalstrom, um anfängliche lokale Minima zu identifizieren;
verwerfen (112) anfänglicher lokaler Minima, die von mindestens einem unmittelbar vorangehenden und/oder unmittelbar folgenden Tal um weniger als einen voreingestellten Schwellenwert abweichen; und
bezeichnen von verbleibenden anfänglichen lokalen Minima als signifikante lokale Minima.

2. Computerimplementiertes Verfahren zum Überwachen von Atmung nach Anspruch 1, wobei ein Bestimmen der Anzahl von Atmungsanstrengungen in dem vorbestimmten Zeitraum ferner ein Zählen eines verpassten Einsetzens von Einatmungen, eines verpassten Einsetzens von Ausatmungen oder beider umfasst;
wobei bestimmt wird, dass ein verpasstes Einsetzen einer Ausatmung aufgetreten ist, wenn (a) zwei oder mehrere signifikante lokale Minima auftreten, ohne dass dazwischen ein signifikantes lokales Maximum auftritt, oder (b) ein lokales Maximum zwischen zwei lokalen Minima auftritt und das lokale Maximum von jedem lokalen Minimum um mehr als eine voreingestellte Zeitspanne getrennt ist, und
wobei bestimmt wird, dass ein verpasstes Einsetzen einer Einatmung aufgetreten ist, wenn (a) zwei oder mehrere signifikante lokale Maxima auftreten, ohne dass ein signifikantes lokales Minimum zu einem Zeitpunkt dazwischen auftritt, oder (b) ein lokales Minimum zwischen zwei lokalen Maxima auftritt und das lokale Minimum von jedem lokalen Maximum um mehr als eine voreingestellte Zeitspanne getrennt ist.

3. Computerimplementiertes Verfahren zum Überwachen von Atmung nach Anspruch 1, ferner umfassend ein Bestimmen der Anzahl von Beatmungen des Individuums während des vorbestimmten Zeitraums.

4. Computerimplementiertes Verfahren nach Anspruch 3, wobei die Anzahl von Beatmungen durch Analysieren eines Thermistorsignalstroms bestimmt wird, der aus einem Nasenluftstrom des Individuums während des vorbestimmten Zeitraums durch ein Verfahren erlangt wird, umfassend ein Identifizieren (107, 109) signifikanter monotoner Abnahmen in dem Thermistorsignalstrom, die für eine längere Zeit als eine voreingestellte Zeitperiode auftreten, und wobei die Anzahl von Beatmungen in dem vorbestimmten Zeitraum die Anzahl signifikanter monotoner Abnahmen in dem Thermistorsignal während des vorbestimmten Zeitraums ist, wobei ein Identifizieren signifikanter monotoner Abnahmen in dem Thermistorsignal Folgendes umfasst:
identifizieren (109) anfänglicher monotoner Abnahmen in dem Thermistorsignalstrom, die länger als eine voreingestellte Zeitspanne auftreten;
verwerfen (111) der anfänglichen monotonen Abnahmen, die eine Amplitudenabnahme von weniger als einem voreingestellten Wert aufweisen; und
bezeichnen der verbleibenden anfänglichen monotonen Abnahmen als signifikante monotone Abnahmen.

5. Computerimplementiertes Verfahren nach Anspruch 1, wobei, wenn die Anzahl von Atmungsanstrengungen niedriger als ein vorbestimmter Wert ist, eine vorbestimmte Aktion durchgeführt wird, eine solche vorbestimmte Aktion umfassend mindestens eines von einem Auslösen eines Alarms oder Aufwecken des Individuums.

6. Computerimplementiertes Verfahren nach Anspruch 1, wobei die Größe der signifikanten lokalen Maxima, der signifikanten lokalen Minima oder beider ausgewertet wird, um die Größe der Atmungsanstrengungen in dem vorbestimmten Zeitraum zu bestimmen.

7. Computerimplementierte Verfahren nach Anspruch 4 ferner umfassend ein Bestimmen von Zeitintervallen zwischen signifikanten monotonen Abnahmen des Thermistorsignals, durch:
Bestimmen eines Amplitudenminimums für jede signifikante monotone Abnahme in dem vorbestimmten Zeitraum;
Messen des Zeitintervalls (a) zwischen jedem aufeinanderfolgenden Amplitudenminimum von jeder signifikanten monotonen Abnahme, (b) zwischen dem Beginn des vorbestimmten Zeitraums und dem ersten Amplitudenminimum; und (c) zwischen dem letzten Amplitudenminimum und dem Ende des vorbestimmten Zeitraums, um dadurch die Zeitintervalle zwischen signifikanten monotonen Abnahmen in dem Thermistorsignal zu bestimmen.

8. Computerlesbares Medium, das computerausführbare Anweisungen speichert, die, wenn sie durch eine Signalverarbeitungsvorrichtung interpretiert und ausgeführt werden, die Signalverarbeitungsvorrichtung veranlassen, das computerimplementierte Verfahren nach einem der Ansprüche 1 bis 7 auszuführen.

9. Signalverarbeitungsvorrichtung, die konfiguriert ist, um das computerimplementierte Verfahren nach einem der Ansprüche 1 bis 7 auszuführen.

## Revendications

1. Procédé mis en œuvre par ordinateur de surveillance de la respiration chez un individu consistant à
obtenir (102) un flux de signal PPG à partir d'un site source central de l'individu;
identifier (108) des pics et des creux du flux de signal PPG à une époque prédéterminée, dans lequel un temps entre des pics ou un temps entre des creux correspond à une fréquence cardiaque de l'individu;
déterminer (110, 112) des maxima locaux significatifs d'amplitudes de pic et des minima locaux significatifs d'amplitudes de creux au sein de l'époque prédéterminée, dans lequel chaque maxima local significatif d'amplitude de pic correspond à un début de l'effort respiratoire de l'individu pour expirer et chaque minima local significatif d'amplitude de creux correspond à un début de l'effort respiratoire de l'individu pour inspirer; calculer (114) le nombre de maxima locaux significatifs, de minima locaux significatifs, ou des deux, pendant l'époque prédéterminée pour déterminer le nombre d'efforts respiratoires dans l'époque prédéterminée; et afficher (116) le nombre d'efforts respiratoires dans l'époque prédéterminée sur un écran d'ordinateur, **caractérisé en ce que** déterminer des maxima locaux significatifs d'amplitudes de pic consiste à déterminer une amplitude de chaque pic dans le flux de signal PPG pendant l'époque prédéterminée;
déterminer une augmentation ou une diminution des pics successifs dans le flux de signal PPG pour identifier les maxima locaux initiaux;
rejeter (112) des maxima locaux initiaux qui s'écartent en amplitude d'au moins un pic immédiatement précédent et/ou immédiatement suivant de moins d'un seuil prédéfini; et
désigner des maxima locaux initiaux restants comme maxima locaux significatifs, et dans lequel la détermination de minima locaux significatifs d'amplitudes de creux consiste à déterminer une amplitude de chaque creux dans le flux de signal PPG pendant l'époque prédéterminée;
déterminer une augmentation ou une diminution des creux successifs dans le flux de signal PPG pour identifier les minima locaux initiaux;
rejeter (112) des minima locaux initiaux qui s'écartent d'au moins un creux immédiatement précédent et/ou immédiatement suivant de moins d'un seuil prédéfini ; et désigner des minima locaux initiaux restants comme des minima locaux significatifs.

2. Procédé mis en œuvre par ordinateur de surveillance de respiration selon la revendication 1, dans lequel la détermination du nombre d'efforts respiratoires dans l'époque prédéterminée consiste en outre à compter le début manqué d'inspirations, le début manqué d'expirations, ou les deux;
dans lequel un début manqué d'expiration est déterminé comme s'étant produit lorsque (a) deux minima locaux significatifs ou plus se produisent sans qu'un maxima local significatif se produit à un moment entre eux, ou (b) un maxima local se produit entre deux minima locaux et le maxima local est séparé de chaque minima local par plus d'un temps prédéfini, et
dans lequel un début manqué de détection d'inspiration est déterminé comme s'étant produit lorsque (a) deux maxima locaux significatifs ou plus se produisent sans qu'un minima local significatif se produit à un moment entre eux, ou (b) un minima local se produit entre deux maxima locaux et le minima local est séparé de chaque maxima local par plus d'un temps prédéfini.

3. Procédé mis en œuvre par ordinateur de surveillance de respiration selon la revendication 1, consistant en outre à déterminer le nombre de ventilations de l'individu pendant l'époque prédéterminée.

4. Procédé mis en œuvre par ordinateur selon la revendication 3, dans lequel le nombre de ventilations est déterminé en analysant un flux de signal de thermistance obtenu à partir du flux d'air nasal de l'individu pendant l'époque prédéterminée par un procédé consistant à identifier (107, 109) des diminutions monotones significatives du flux de signal de thermistance qui se produisent pendant un temps supérieur à une période de temps prédéfinie, et dans lequel le nombre de ventilations dans l'époque prédéterminée est le nombre de diminutions monotones significatives du signal de thermistance pendant l'époque prédéterminée, dans lequel l'identification de diminutions monotones significatives dans le signal de thermistance consiste à:
identifier (109) des diminutions monotones initiales dans le flux de signal de thermistance qui se produisent pendant un temps supérieur à une période de temps prédéfinie;
rejeter (111) les diminutions monotones initiales ayant une diminution d'amplitude inférieure à une valeur prédéfinie; et
désigner les diminutions monotones initiales restantes comme des diminutions monotones significatives.

5. Procédé mis en œuvre par ordinateur selon la revendication 1, dans lequel si le nombre d'efforts respiratoires est inférieur à une valeur prédéterminée, une action prédéterminée est effectuée, une telle action prédéterminée comprenant au moins un parmi le déclenchement d'une alarme ou le réveil de l'individu.

6. Procédé mis en œuvre par ordinateur selon la revendication 1, dans lequel l'amplitude des maxima locaux significatifs, des minima locaux significatifs ou des deux sont évaluée pour déterminer l'amplitude des efforts respiratoires à l'époque prédéterminée.

7. Procédé mis en œuvre par ordinateur selon la revendication 4, consistant en outre à déterminer des intervalles de temps entre des diminutions monotones significatives du signal de thermistance, en:
Déterminant un minimum d'amplitude pour chaque diminution monotone significative dans l'époque prédéterminée;
mesurer l'intervalle de temps (a) entre chaque minimum d'amplitude successif de chaque diminution monotone significative, (b) entre le début de l'époque prédéterminée et le premier minimum d'amplitude; et (c) entre le dernier minimum d'amplitude et la fin de l'époque prédéterminée, pour ainsi déterminer les intervalles de temps entre des diminutions monotones significatives du signal de thermistance.

8. Support lisible par ordinateur qui stocke des instructions exécutables par ordinateur qui, lorsqu'elles sont interprétées et exécutées par un dispositif de traitement de signal, amènent le dispositif de traitement de signal à mettre en œuvre le procédé mis en œuvre par ordinateur selon l'une quelconque des revendications 1 à 7.

9. Dispositif de traitement de signal configuré pour mettre en œuvre le procédé mis en œuvre par ordinateur selon l'une quelconque des revendications 1 à 7.
